# EUROPÄISCHE PATENTSCHRIFT

(11) **EP 3 315 154 B1**
(45) Veröffentlichungstag und Bekanntmachung des Hinweises auf die Patenterteilung: **07.07.2021**
(21) Anmeldenummer: 16196318.6
(22) Anmeldetag: 28.10.2016
(51) Int. Cl.: A61M 5/315, F16H 25/20

(54) **ZWEISTUFIGER TELESKOPSPINDELANTRIEB**
TWO-STAGE TELESCOPIC SPINDLE DRIVE
ENTRAÎNEMENT DE BROCHE TÉLESCOPIQUE BIÉTAGÉ

(43) Veröffentlichungstag der Anmeldung: 02.05.2018
(73) Patentinhaber: maxon international ag, 6072 Sachseln (CH)
(72) Erfinder: Horn, Carsten, 77955 Ettenheim (CH)
(74) Vertreter: Grünecker Patent- und Rechtsanwälte PartG mbB

(56) Entgegenhaltungen:
- WO-A1-2011/006925
- WO-A1-2015/172828
- WO-A1-2015/172962
- DE-A1- 19 717 107
- DE-T2- 69 502 474
- US-A1- 2004 000 818
- US-A1- 2010 018 334
- US-A1- 2014 290 403

## Beschreibung

Die vorliegende Erfindung betrifft einen Teleskopspindelantrieb nach dem Oberbegriff des unabhängigen Anspruchs 1.

Ein gattungsgemäßer Teleskopspindelantrieb weist ein erstes Teleskopelement, ein gegenüber dem ersten Teleskopelement entlang einer Achse des Teleskopspindelantriebs ausfahrbares zweites Teleskopelement, und ein gegenüber dem zweiten Teleskopelement entlang der Achse ausfahrbares drittes Teleskopelement auf. Der Antrieb des Teleskopspindelantriebs weist ein rotativ angetriebenes erstes Antriebselement auf, das drehbar und axial unverschieblich gegenüber dem ersten Teleskopelement gelagert ist. Ein Gewinde des ersten Antriebselements und ein damit in Eingriff befindliches erstes Gewinde des zweiten Teleskopelements bilden zusammen einen ersten Spindeltrieb. Ein zweites Gewinde des zweiten Teleskopelements und ein damit in Eingriff befindliches Gewinde des dritten Teleskopelements bilden zusammen einen zweiten Spindeltrieb. Der Antrieb des Teleskopspindelantriebs weißt ein rotativ angetriebenes zweites Antriebselement auf, das ebenfalls drehbar gegenüber dem ersten Teleskopelement gelagert ist, wobei zwischen dem zweiten Antriebselement und dem zweiten Teleskopelement ein rotativ mitnehmender Eingriff besteht, und wobei das zweite Teleskopelement gegenüber dem zweiten Antriebselement in axialer Richtung verschieblich ist.

Ein Teleskopspindelantrieb gemäß dem Oberbegriff ist beispielsweise aus der Entgegenhaltung DE 695 02 474 T2 bekannt.

Teleskopspindelantriebe werden beispielsweise in der Medizintechnik bei Medikamentendosiersystemen oder Injektionsvorrichtungen eingesetzt. Hierbei handelt es sich in der Regel um kleine, tragbare, Batterie- oder Akku-betriebene Handgeräte. Ein üblicher Teleskopspindelantrieb für derartige Anwendungen ist beispielsweise in WO201106925 A1 beschrieben. Ein Teleskopspindelantrieb der gattungsgemäßen Art nach dem Oberbegriff des unabhängigen Anspruchs 1 ist in der DE 19717107 A1 beschrieben. Bei diesem Teleskopspindelantrieb wird das zweite Teleskopelement in Abhängigkeit der auf das zweite Teleskopelement wirkenden Reibungskräfte entweder drehend mitgenommen oder durch den ersten Spindeltrieb entlang der Achse des Teleskopspindelantriebs verschoben. Die Bewegung des zweiten Teleskopelements kann auch eine zusammengesetzte Verschiebe-Drehbewegung sein. Sofern das zweite Teleskopelement verschoben wird, nimmt es das dritte Teleskopelement mit. Soweit das zweite Teleskopelement verdreht wird, erzeugt die Drehbewegung über den zweiten Spindeltrieb eine Vorschubbewegung des dritten Teleskopelements.

Bei den zuvor genannten Anwendungen bekannter Teleskopspindelantriebe kommt es in Bezug auf die Bedienerfreundlichkeit vornehmlich darauf an, dass die Laufzeit des Geräts, in welchem der Teleskopspindelantrieb zum Einsatz kommt, möglichst lang ist. Gleichzeitig sollen die Antriebe auch schnell und leistungsfähig sein.

Es ist Aufgabe der vorliegenden Erfindung, den Teleskopspindelantrieb der gattungsgemäßen Art in vorteilhafter Weise weiterzubilden und dabei den Wirkungsgrad zu erhöhen.

Die Aufgabe wird gelöst durch die Merkmale des unabhängigen Anspruchs 1. Demnach liegt bei einem Teleskopspindelantrieb der gattungsgemäßen Art gemäß dem Oberbegriff des unabhängigen Anspruchs 1 dann eine erfindungsgemäße Lösung der Aufgabe vor, wenn das erste Antriebselement und das zweite Antriebselement als zwei ineinander gesetzte Hülsen ausgeführt sind. Dabei umschließt das erste Antriebselement das zweite Antriebselement. Diese trägt zu einer kompakten Bauweise des erfindungsgemäßen Teleskopspindelantriebs bei. Außerdem weist das zweite Teleskopelement eine äußere Hülse und einen von der äußeren Hülse des zweiten Teleskopelements umschlossenen und fest mit dieser verbundenen mittleren Fortsatz auf, wobei erstes und zweites Antriebselement in einen Zwischenraum zwischen der äußeren Hülse des zweiten Teleskopelements und dem mittleren Fortsatz eintauchen, wobei das Gewinde des ersten Antriebselement ein Außengewinde ist, und wobei das erste Gewinde des zweiten Teleskopelements ein Innengewinde ist, das an einer Innenseite der äußeren Hülse des zweiten Teleskopelements ausgebildet ist. Der mittlere Fortsatz des zweiten Teleskopelements kann beispielsweise einen 4-eckigen Querschnitt aufweisen und in einer ebenfalls 4-eckigen Ausnehmung des zweiten Antriebselements axial verschieblich geführt sein. Dadurch wird eine einfache Verdrehsicherung zwischen dem zweiten Teleskopelement und dem zweiten Antriebselement erreicht. Selbstverständlich sind auch alternative Querschnitte denkbar, die einen entsprechenden Formschluss zur axialen Führung gewährleisten.

Gegenüber dem aus DE 19717107 A1 bekannten Teleskopspindelantrieb hat die erfindungsgemäße Lösung den Vorteil, dass es nicht mehr von den wirkenden Reibungskräften abhängt, ob das zweite Teleskopelement lediglich mitgedreht oder axial verschoben wird. Die Vorschubgeschwindigkeit sowohl des zweiten Teleskopelements als auch des dritten Teleskopelements kann durch die Geschwindigkeitsdifferenz der Drehgeschwindigkeiten des ersten und zweiten Antriebselements eingestellt werden. Dadurch lässt sich der Antrieb insgesamt auch in Bezug auf die wirkenden Reibungskräfte optimieren.

Der Teleskopspindelantrieb kann eine Basis aufweisen, die entweder fest mit dem ersten Teleskopelement verbunden ist oder selbst das erste Teleskopelement bildet. Die beiden Antriebselemente sind vorzugsweise an der Basis drehbar gelagert.

Vorteilhafte Weiterbildungen der vorliegenden Erfindung sind Gegenstand der Unteransprüche.

Gemäß einer besonders bevorzugten Ausführungsform der vorliegenden Erfindung ist der Antrieb des Teleskopspindelantriebs derart eingerichtet, dass die Rotationsgeschwindigkeit des ersten Antriebselements doppelt so groß ist wie die Rotationsgeschwindigkeit des zweiten Antriebselements. Vorzugsweise haben das erste Antriebselement und das zweite Antriebselement die gleiche Drehrichtung. Die axiale Geschwindigkeit des dritten Teleskopelements ist bei dieser Ausführungsform doppelt so groß wie die axiale Geschwindigkeit des zweiten Teleskopelements. Bei einheitlicher Gewindesteigung der in Eingriff befindlichen Gewinde legt das dritte Teleskopelement doppelt so viel Wegstrecke zurück wie das zweite Teleskopelement. Dies hat zur Folge, dass sich alle Gewindebauteile nur mit der einfachen Antriebsgeschwindigkeit, die der Drehgeschwindigkeit des zweiten Antriebselements entspricht, relativ zueinander bewegen. Dadurch wird die Reibung minimiert und der Wirkungsgrad optimiert. Auch das benötigte Drehmoment wird auf diese Weise optimal eingestellt. Ferner wird die Lebensdauer des Teleskopspindelantrieb dadurch verbessert, und es können große Axialkräfte erzielt werden.

Selbstverständlich ist es auch möglich, für das Verhältnis der Drehgeschwindigkeiten des ersten und zweiten Antriebselements ein anderes Verhältnis zu wählen. Es ist jedoch im Sinne der vorliegenden Erfindung, dass das erste Antriebselement mit einer anderen Geschwindigkeit angetrieben wird wie das zweite Antriebselement.

Durch alternative Kombinationen der Rotationsgeschwindigkeiten der beiden Antriebselemente, sowie unterschiedliche Gewindesteigungen und Gewindelängen kann der Teleskopspindelantrieb variabel an spezielle Anforderungen angepasst werden.

Gemäß einer weiteren besonders bevorzugten Ausführungsform der vorliegenden Erfindung sind das erste Antriebselement und das zweite Antriebselement koaxial zueinander und jeweils koaxial zur Achse des Teleskopspindelantrieb drehbar und axial unverschieblich gegenüber dem ersten Teleskopelement gelagert. Diese Ausführungsform gewährleistet eine besonders kompakte Bauweise. Dabei ist es von besonderem Vorteil, wenn das erste Antriebselement ein erstes Ritzel aufweist, wobei das zweite Antriebselement ein zweites Ritzel aufweist, und wobei erstes und zweites Antriebselement über erstes und zweites Ritzel mittels eines Stirnradgetriebes des Teleskopspindelantriebs von einem einzigen Elektromotor antreibbar sind. Dadurch ergibt sich nicht nur eine kompakte sondern auch eine besonders kostengünstige Bauweise.

Das Verhältnis der Antriebsgeschwindigkeiten des ersten und zweiten Antriebselements ist durch das Stirnradgetriebe unveränderlich festgelegt. Der Elektromotor ist vorzugsweise ebenfalls Bestandteil des erfindungsgemäßen Teleskopspindelantriebs. In einer alternativen Ausführungsform ist es auch denkbar, die beiden Antriebselemente jeweils separat mittels zweier unabhängiger Elektromotoren anzutreiben. Der Geschwindigkeitsunterschied zwischen den beiden Antriebselementen kann dabei individuell eingestellt oder auch geregelt werden.

Gemäß einer weiteren bevorzugten Ausführungsform der vorliegenden Erfindung ist das zweite Gewinde des zweiten Teleskopelements ein Außengewinde, das an einer Außenseite der äußeren Hülse des zweiten Teleskopelements ausgebildet ist. Dieses Außengewinde wirkt mit einem entsprechenden Innengewinde des dritten Teleskopelements zusammen.

Gemäß einer weiteren bevorzugten Ausführungsform der vorliegenden Erfindung ist das dritte Teleskopelement als Plungerhülse ausgebildet.

Gemäß einer weiteren besonders bevorzugten Ausführungsform der vorliegenden Erfindung ist das dritte Teleskopelement gegenüber dem ersten Teleskopelement gegen Verdrehung gesichert. Dabei ist das erste Teleskopelement weiter vorzugsweise als feststehende Hülse ausgeführt, wobei der Teleskopspindelantrieb ferner eine mittlere Hülse aufweist, die das zweite Teleskopelement zumindest teilweise radial umschließt und gegenüber dem als feststehende Hülse ausgeführten ersten Teleskopelement entlang der Achse des Teleskopspindelantrieb verschieblich gelagert ist, wobei das dritte Teleskopelement ebenfalls als Hülse ausgeführt und gegenüber der mittleren Hülse entlang der Achse des Teleskopspindelantrieb verschieblich gelagert ist, und wobei sowohl zwischen dem ersten Teleskopelement und der mittleren Hülse als auch zwischen der mittleren Hülse und dem dritten Teleskopelement eine Verdrehsicherung besteht. Die Verdrehsicherung wird weiter vorzugsweise mittels zumindest eines Zapfens realisiert, der an einer Hülse ausgebildet ist und in einem Führungsschlitz oder einer Führungsnut der jeweils anderen Hülse axial geführt ist. Alle drei Hülsen, nämlich das erste Teleskopelement, die mittlere Hülse und das dritte Teleskopelement, sind koaxial zueinander angeordnet und tauchen ineinander ein.

In einer weiteren bevorzugten Ausführungsform der vorliegenden Erfindung ist das erste Teleskopelement das äußerste Teleskopelement des Teleskopspindelantriebs und weist dabei einen Außendurchmesser kleiner 50 mm, vorzugsweise kleiner 25 mm auf.

Alle Bauteile der Spindelmechanik bestehen vorzugsweise aus Metall und/oder Keramik. Insbesondere sind sämtliche Bauteile des Teleskopspindelantriebs, die ein Gewinde aufweisen, vorzugsweise aus einer oberflächenpolierten Keramik, insbesondere aus einer Zirkonium- oder Aluminiumoxidkeramik gefertigt. Der erfindungsgemäße Teleskopspindelantrieb ist dadurch besonders kostengünstig und weist eine hohe Lebensdauer auf.

Ein Ausführungsbeispiel der vorliegenden Erfindung wird im Folgenden anhand von Zeichnungen näher erläutert.

Es zeigen:
- Figur 1:: ein Ausführungsbeispiel eines erfindungsgemäßen Teleskopspindelantriebs in einer Seitenansicht,
- Figur 2:: einen Längsschnitt des erfindungsgemäßen Teleskopspindelantriebs aus Figur 1, und
- Figur 3:: den erfindungsgemäßen Teleskopspindelantrieb aus den Figuren 1 und 2 im Querschnitt entlang der in Figur 2 eingezeichneten Schnittlinie III.

Für die folgenden Ausführungen gilt, dass gleiche Teile durch gleiche Bezugszeichen bezeichnet sind. Sofern in einer Figur Bezugszeichen enthalten sind, auf die in der zugehörigen Figurenbeschreibung nicht näher eingegangen wird, so wird auf vorangehende oder nachfolgende Figurenbeschreibungen Bezug genommen.

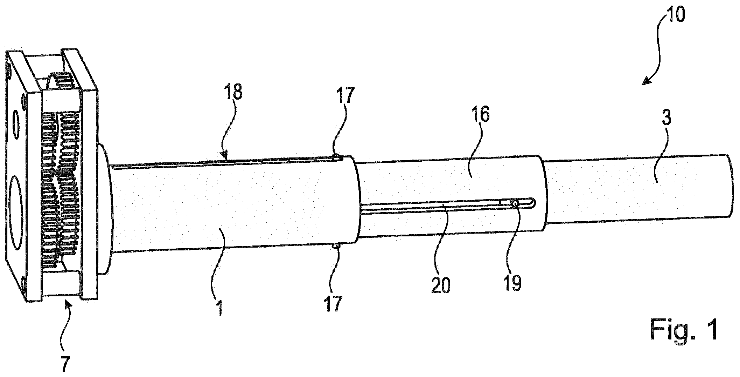

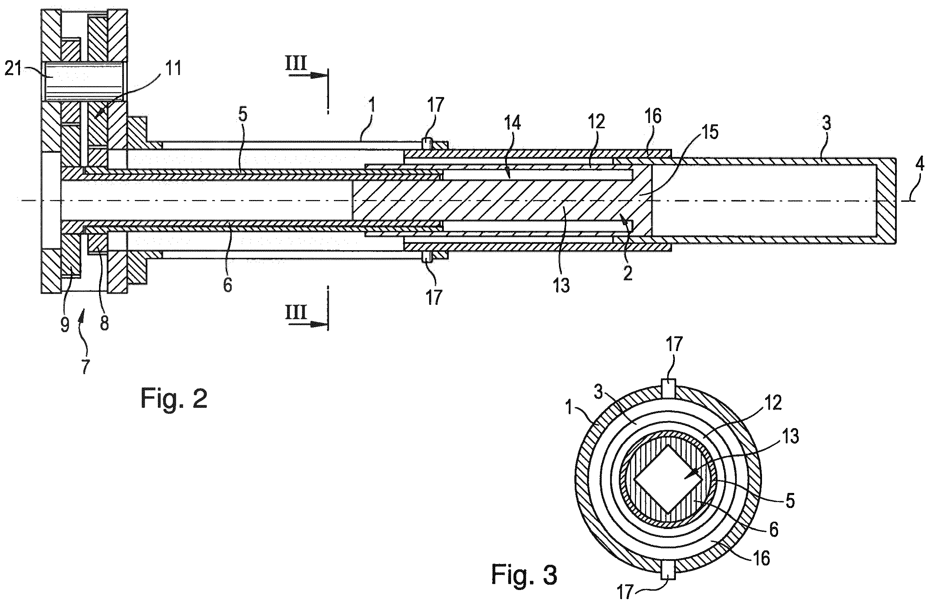

Figur 1 zeigt ein Ausführungsbeispiel des erfindungsgemäßen Teleskopspindelantriebs 10 in einer Seitenansicht. Die Darstellung zeigt den Teleskopspindelantrieb in der maximal ausgefahrenen Stellung. Zu erkennen sind die feststehende Basis 7, das fest mit der Basis verbundene erste Teleskopelement 1 und das vollständig ausgefahrene dritte Teleskopelement 3 des erfindungsgemäßen Teleskopspindelantriebs. Das zweite Teleskopelement 2 ist hingegen lediglich in der Schnittdarstellung aus Figur 2 gezeigt. In Figur 1 ist es verdeckt durch eine zwischen dem ersten Teleskopelement und dem dritten Teleskopelement angeordnete mittlere Hülse 16. Die mittlere Hülse 16 dient vorrangig der Verdrehsicherung des dritten Teleskopelements. Zu diesem Zweck weist das dritte Teleskopelement 3 zwei Zapfen 19 auf, die in entsprechenden Längsschlitzen 20 der mittleren Hülse axial geführt sind und das dritte Teleskopelement gegenüber der mittleren Hülse gegen Verdrehung sichern. Gleichermaßen weist die mittlere Hülse ebenfalls zwei Zapfen 17 auf, die in entsprechenden Längsschlitzen 18 des ersten Teleskopelements 1 axial geführt sind und die mittlere Hülse gegenüber dem ersten Teleskopelement gegen Verdrehung sichern. Das erste Teleskopelement und das dritte Teleskopelement sind, ebenso wie die mittlere Hülse 16, gleichermaßen als Hülsen ausgebildet, sodass die Bauteile 1, 16 und 3 nach Art eines Teleskops ineinandergesetzt sind und ineinander eintauchen, wenn der erfindungsgemäße Teleskopspindelantrieb eingefahren wird.

Der Längsschnitt aus Figur 2 zeigt das mechanische Innenleben des erfindungsgemäßen Teleskopspindelantriebs 10. Zunächst ist zu erkennen, dass die bereits zuvor genannten Bauteile 1, 16 und 3 koaxial zur Achse 4 des erfindungsgemäßen Teleskopspindelantriebs angeordnet sind. Ebenfalls koaxial zur Achse 4 sind ferner zwei Antriebselemente 5 und 6 angeordnet. Die Antriebselemente 5 und 6 sind in der Basis 7 des erfindungsgemäßen Teleskopspindelantriebs drehbar und axial unverschieblich gelagert. Beide Antriebselemente sind als Hülsen ausgeführt, wobei das erste Antriebselement 5 das zweite Antriebselement 6 radial umschließt. Der Teleskopspindelantrieb verfügt über eine Antriebssektion, die an der Basis 7 angebracht ist und beim gezeigten Ausführungsbeispiel ein Stirnradgetriebe 11 aufweist, dessen Eingangswelle 21 durch einen nicht gezeigten Elektromotor angetrieben wird. Das Stirnradgetriebe 11 steht sowohl mit einem Ritzel 8 des ersten Antriebselements 5 als auch mit einem Ritzel 9 des zweiten Antriebselements 6 in Eingriff. Das Übersetzungsverhältnis ist dabei so gewählt, dass sich das erste Antriebselement 5 doppelt so schnell dreht wie das zweite Antriebselement 6.

Beide Antriebselemente 5 und 6 stehen in Eingriff mit dem zweiten Teleskopelement 2 des erfindungsgemäßen Teleskopspindelantriebs. Dieses besteht aus einer äußeren Hülse 12 und einem von der äußeren Hülse 12 umschlossenen Fortsatz 13, der über das Kopfende 15 des zweiten Teleskopelements fest oder auch einstückig mit der äußeren Hülse 12 verbunden ist. Zwischen der äußeren Hülse 12 und dem mittleren Fortsatz 13 besteht ein ringförmiger Zwischenraum 14, in welchem sich sowohl das hülsenförmige erste Antriebselement 5 als auch das von diesem umschlossene, hülsenförmige zweite Antriebselement 6 befinden. Die Antriebselemente 5 und 6 tauchen umso mehr in den Zwischenraum 14 ein, je weiter der erfindungsgemäße Teleskopspindelantrieb eingefahren wird. Das erste Antriebselement verfügt über ein Außengewinde, das mit einem Innengewinde in Eingriff steht, welches an der Innenseite der äußeren Hülse 12 des zweiten Teleskopelements 2 ausgebildet ist. Das Außengewinde des ersten Antriebselements und das Innengewinde an der Innenseite der äußeren Hülse 12 bilden zusammen einen ersten Spindeltrieb des erfindungsgemäßen Teleskopspindelantriebs. Das zweite Teleskopelement 2 verfügt gleichermaßen über ein Außengewinde, das an der Außenseite der äußeren Hülse 12 ausgebildet ist und mit einem entsprechenden Innengewinde des dritten Teleskopelements 3 in Eingriff steht. Das Außengewinde des zweiten Teleskopelements und das Innengewinde des dritten Teleskopelements bilden zusammen einen zweiten Spindeltrieb des erfindungsgemäßen Teleskopspindelantriebs.

Wie insbesondere aus Figur 3 deutlich wird, besteht zwischen dem Fortsatz 13 des zweiten Teleskopelements 2 und einer entsprechenden Ausnehmung des zweiten Antriebselements ein Formschluss, der dafür sorgt, dass sich das zweite Teleskopelement 2 mit dem zweiten Antriebselement 6 mitdreht. Das zweite Teleskopelement 2 ist gegenüber dem zweiten Antriebselement 6 jedoch axial verschieblich. Der Fortsatz 13 des zweiten Teleskopelements weist zu diesem Zweck einen 4-eckigen Querschnitt auf, die Ausnehmung in dem zweiten Antriebselement 6 ist entsprechend ebenfalls 4-eckig. Selbstverständlich sind andere Querschnitte denkbar, die einen Formschluss zwischen den beiden Bauteilen gewährleisten und dabei gleichzeitig eine axiale Bewegung des zweiten Teleskopelements 2 gegenüber dem zweiten Antriebselement 6 erlauben.

Die nicht dargestellten Gewinde der angesprochenen Bauteile sind bei dem Ausführungsbeispiel alle mit gleicher Steigung und alle mit gleicher Drehrichtung ausgeführt. Dadurch, dass sich das erste Antriebselement 5 etwa doppelt so schnell dreht wie das zweite Antriebselement 6, wird aufgrund der Verdrehsicherung des dritten Teleskopelements gegenüber dem ersten Teleskopelement ein Vorschub des dritten Teleskopelements erzeugt, der doppelt so groß ist wie der Vorschub des zweiten Teleskopelements. Dabei entspricht die relative Rotationsgeschwindigkeit sämtlicher Gewindebauteile zueinander der einfachen Antriebsgeschwindigkeit des zweiten Antriebselements, wodurch der Wirkungsgrad des erfindungsgemäßen Teleskopspindelantriebs optimiert wird.

## Patentansprüche

1. Teleskopspindelantrieb (10) mit einem ersten Teleskopelement (1), einem gegenüber dem ersten Teleskopelement (1) entlang einer Achse (4) des Teleskopspindelantriebs (10) ausfahrbaren zweiten Teleskopelement (2), und einem gegenüber dem zweiten Teleskopelement (2) entlang der Achse (4) ausfahrbaren dritten Teleskopelement (3), wobei ein Antrieb des Teleskopspindelantriebs (10) ein rotativ angetriebenes erstes Antriebselement (5) aufweist, das drehbar und axial unverschieblich gegenüber dem ersten Teleskopelement (1) gelagert ist, wobei ein Gewinde des ersten Antriebselements (5) und ein damit in Eingriff befindliches erstes Gewinde des zweiten Teleskopelements (2) zusammen einen ersten Spindeltrieb bilden, und wobei ein zweites Gewinde des zweiten Teleskopelements (2) und ein damit in Eingriff befindliches Gewinde des dritten Teleskopelements (3) zusammen einen zweiten Spindeltrieb bilden, wobei der Antrieb des Teleskopspindelantriebs (10) ein rotativ angetriebenes zweites Antriebselement (6) aufweist, das ebenfalls drehbar gegenüber dem ersten Teleskopelement (1) gelagert ist, wobei zwischen dem zweiten Antriebselement (6) und dem zweiten Teleskopelement (2) ein rotativ mitnehmender Eingriff besteht, und wobei das zweite Teleskopelement (2) gegenüber dem zweiten Antriebselement (6) in axialer Richtung verschieblich ist, **dadurch gekennzeichnet, dass** das erstes Antriebselement (5) und das zweites Antriebselement (6) als zwei ineinander gesetzte Hülsen ausgeführt sind, wobei das zweite Teleskopelement (2) eine äußere Hülse (12) und einen von der äußeren Hülse (12) des zweiten Teleskopelements (2) umschlossenen und fest mit dieser verbundenen mittleren Fortsatz (13) aufweist, wobei erstes und zweites Antriebselement (5, 6) in einen Zwischenraum (14) zwischen der äußeren Hülse (12) des zweiten Teleskopelements (2) und dem mittleren Fortsatz (13) eintauchen, wobei das Gewinde des ersten Antriebselements (5) ein Außengewinde ist, und wobei das erste Gewinde des zweiten Teleskopelements (2) ein Innengewinde ist, das an einer Innenseite der äußeren Hülse (12) des zweiten Teleskopelements (2) ausgebildet ist.

2. Teleskopspindelantrieb (10) nach Anspruch 1, **dadurch gekennzeichnet, dass** der Antrieb derart eingerichtet ist, dass die Rotationsgeschwindigkeit des ersten Antriebselements (5) doppelt so groß ist wie die Rotationsgeschwindigkeit des zweiten Antriebselements (6).

3. Teleskopspindelantrieb (10) nach Anspruch 1 oder 2, **dadurch gekennzeichnet, dass** das erste Antriebselement (5) und das zweite Antriebselement (6) koaxial zueinander und jeweils koaxial zur Achse (4) des Teleskopspindelantriebs (10) drehbar und axial unverschieblich gegenüber dem ersten Teleskopelement (1) gelagert sind.

4. Teleskopspindelantrieb (10) nach Anspruch 3, **dadurch gekennzeichnet, dass** das erste Antriebselement (5) ein erstes Ritzel (8) aufweist, wobei das zweite Antriebselement (6) ein zweites Ritzel (9) aufweist, und wobei erstes und zweites Antriebselement über erstes und zweites Ritzel mittels eines Stirnradgetriebes (11) des Teleskopspindelantriebs (10) von einem einzigen Elektromotor antreibbar sind.

5. Teleskopspindelantrieb (10) nach einem der Ansprüche 1 bis 4, **dadurch gekennzeichnet, dass** das zweite Gewinde des zweiten Teleskopelements (2) ein Außengewinde ist, das an einer Außenseite der äußeren Hülse (12) des zweiten Teleskopelements (2) ausgebildet ist.

6. Teleskopspindelantrieb (10) nach einem der Ansprüche 1 bis 5, **dadurch gekennzeichnet, dass** das dritte Teleskopelement (3) als Plungerhülse ausgebildet ist.

7. Teleskopspindelantrieb (10) nach einem der Ansprüche 1 bis 6, **dadurch gekennzeichnet, dass** das dritte Teleskopelement (3) gegenüber dem ersten Teleskopelement (1) gegen Verdrehung gesichert ist.

8. Teleskopspindelantrieb (10) nach Anspruch 7, **dadurch gekennzeichnet, dass** das erste Teleskopelement (1) als feststehende Hülse ausgeführt ist, wobei der Teleskopspindelantrieb (10) ferner eine mittlere Hülse (16) aufweist, die das zweite Teleskopelement (2) zumindest teilweise radial umschließt und gegenüber dem als feststehende Hülse ausgeführten ersten Teleskopelement (1) entlang der Achse (4) des Teleskopspindelantriebs (10) verschieblich gelagert ist, wobei das dritte Teleskopelement (3) ebenfalls als Hülse ausgeführt und gegenüber der mittleren Hülse (16) entlang der Achse (4) des Teleskopspindelantriebs (10) verschieblich gelagert ist, und wobei sowohl zwischen dem ersten Teleskopelement (1) und der mittleren Hülse (16) als auch zwischen der mittleren Hülse (16) und dem dritten Teleskopelement (3) eine Verdrehsicherung besteht.

9. Teleskopspindelantrieb (10) nach einem der Ansprüche 1 bis 8, **dadurch gekennzeichnet, dass** das erste Teleskopelement (1) das äußerste Teleskopelement des Teleskopspindelantriebs (10) ist und einen Außendurchmesser kleiner 50 mm, vorzugsweise kleiner 25 mm aufweist.

10. Teleskopspindelantrieb (10) nach einem der Ansprüche 1 bis 9, **dadurch gekennzeichnet, dass** sämtliche Bauteile des Teleskopspindelantriebs (10), die ein Gewinde aufweisen, aus einer oberflächenpolierten Keramik, insbesondere aus einer Zirkonium- oder AluminiumoxidKeramik gefertigt sind.

## Claims

1. Telescopic spindle drive (10) with a first telescopic element (1), a second telescopic element (2) extendable along an axis (4) of the telescopic spindle drive (10) with respect to the first telescopic element (1), and a third telescopic element (3) extendable along the axis (4) with respect to the second telescopic element (2), wherein the drive of the telescopic spindle drive (10) includes a first drive element (5) driven rotatably, which is mounted rotatably and non-slidably in axial direction with respect to the first telescopic element (1), wherein a thread of the first drive element (5) and a therewith engaged first thread of the second telescopic element (2) jointly constitute a first spindle drive, and wherein a second thread of the second telescopic element (2) and a therewith engaged thread of the third telescopic element (3) jointly constitute a second spindle drive, wherein the drive of the telescopic spindle drive (10) includes a second, rotatably driven drive element (6) that is also mounted rotatably with respect to the first telescopic element (1), wherein there is a rotative driven engagement between the second drive element (6) and the second telescopic element (2), and wherein the second telescopic element (2) is slidable in axial direction with respect to the second drive element (6), **characterised in that** the first drive element (5) and the second drive element (6) are implemented as two nested bushings, wherein the second telescopic element (2) includes an external bushing (12) and an intermediary extension (13) that is enclosed in and firmly attached to the external bushing (12) of the second telescopic element (2), wherein the first and second drive elements (5, 6) are plunged in an interspace (14) between the external bushing (12) of the second telescopic element (2) and the intermediary extension (13), wherein the thread of the first drive element (5) is an external thread, and wherein the first thread of the second telescopic element (2) is an internal thread that is formed on an inner side of the external bushing (12) of the second telescopic element (2).

2. Telescopic spindle drive (10) of claim 1, **characterised in that** the drive is configured so that the rotation speed of the first drive element (5) is twice as high as the rotation speed of the second drive element (6).

3. Telescopic spindle drive (10) of claim 1 or 2, **characterised in that** the first drive element (5) and the second drive element (6) are mounted coaxially with each other as well as coaxially each with the axis (4) of the telescopic spindle drive (10), rotationally and non-slidably in axial direction with respect to the first telescopic element (1).

4. Telescopic spindle drive (10) of claim 3, **characterised in that** the first drive element (5) comprises a first pinion (8), wherein the second drive element (6) comprises a second pinion (9), and wherein the first and second drive elements are drivable by one single electric motor via the first and second pinions by means of a spur wheel gear (11) of the telescopic spindle drive (10).

5. Telescopic spindle drive (10) of one of claims 1 to 4, **characterised in that** the second thread of the second telescopic element (2) is an external thread which is formed on an external side of the external bushing (12) of the second telescopic element (2).

6. Telescopic spindle drive (10) of one of claims 1 to 5, **characterised in that** the third telescopic element (3) is formed as a piston bushing.

7. Telescopic spindle drive (10) of one of claims 1 to 6, **characterised in that** the third telescopic element (3) is secured against twisting with respect to the first telescopic element (1).

8. Telescopic spindle drive (10) of claim 7, **characterised in that** the first telescopic element (1) is implemented as a fixed bushing, wherein the telescopic spindle drive (10) further comprises an intermediary bushing (16) that at least partially encloses the second telescopic element (2) radially and is mounted slidably along the axis (4) of the telescopic spindle drive (10) with respect to the first telescopic element (1) implemented as a fixed bushing, wherein the third telescopic element (3) is also implemented as a bushing and is mounted slidably along the axis (4) of the telescopic spindle drive (10) with respect to the intermediary bushing (16), and wherein an anti-rotation device is present between the first telescopic element (1) and the intermediary bushing (16) as well as between the intermediary bushing (16) and the third telescopic element (3) .

9. Telescopic spindle drive (10) of one of claims 1 to 8, **characterised in that** the first telescopic element (1) is the outermost telescopic element of the telescopic spindle drive (10) and has an outer diameter smaller than 50 mm, and preferably smaller than 25 mm.

10. Telescopic spindle drive (10) of one of claims 1 to 9, **characterised in that** all components of the telescopic spindle drive (10) that feature a thread are manufactured out of surface-polished ceramic, and in particular out of a zirconium oxide or aluminium oxide ceramic.

## Revendications

1. Entraînement à vis télescopique (10) avec un premier élément télescopique (1), un deuxième élément télescopique (2) extensible le long d'un axe (4) de l'entraînement à vis télescopique (10) par rapport au premier élément télescopique (1), et un troisième élément télescopique (3) extensible le long de l'axe (4) par rapport au deuxième élément télescopique (2), dans lequel un entraînement de l'entraînement à vis télescopique (10) comporte un premier élément d'entraînement (5) à entraînement rotatif qui est monté de façon rotative et non coulissante axialement par rapport au premier élément télescopique (1), dans lequel un filetage du premier élément d'entraînement (5) et un premier filetage du deuxième élément télescopique (2) avec lequel il est engrené constituent conjointement un premier actionneur à vis, et dans lequel un deuxième filetage du deuxième élément télescopique (2) et un filetage du troisième élément télescopique (3) avec lequel il est engrené constituent conjointement un deuxième actionneur à vis, dans lequel l'entraînement de l'entraînement à vis télescopique (10) comporte un deuxième élément d'entraînement entraîné en rotation (6) qui est également monté de manière rotative par rapport au premier élément télescopique (1), dans lequel un engagement d'entraînement en rotation est présent entre le deuxième élément d'entraînement (6) et le deuxième élément télescopique (2), et dans lequel le deuxième élément télescopique (2) est coulissant en direction axiale par rapport au deuxième élément d'entraînement (6), **caractérisé en ce que** le premier élément d'entraînement (5) et le deuxième élément d'entraînement (6) sont réalisés comme deux douilles emboîtées, dans lequel le deuxième élément télescopique (2) comporte une douille externe (12) et une extension médiane (13) entourée par la douille externe (12) du deuxième élément télescopique (2) et connectée de manière fixe à celle-ci, dans lequel les premier et deuxième éléments d'entraînement (5, 6) sont plongés dans un espace (14) entre la douille externe (12) du deuxième élément télescopique (2) et l'extension médiane (13), dans lequel le filetage du premier élément d'entraînement (5) est un filetage externe, et dans lequel le premier filetage du deuxième élément télescopique (2) est un filetage interne qui est formé sur un côté interne de la douille externe (12) du deuxième élément télescopique (2).

2. Entraînement à vis télescopique (10) selon la revendication 1, **caractérisé en ce que** l'entraînement est configuré de telle sorte que la vitesse de rotation du premier élément d'entraînement (5) est deux fois plus importante que la vitesse de rotation du deuxième élément d'entraînement (6).

3. Entraînement à vis télescopique (10) selon la revendication 1 ou 2, **caractérisé en ce que** le premier élément d'entraînement (5) et le deuxième élément d'entraînement (6) sont montés de manière rotative et non coulissante axialement par rapport au premier élément télescopique (1) de façon coaxiale entre eux et de façon coaxiale chacun avec l'axe (4) de l'entraînement à vis télescopique (10).

4. Entraînement à vis télescopique (10) selon la revendication 3, **caractérisé en ce que** le premier élément d'entraînement (5) comporte un premier pignon (8), dans lequel le deuxième élément d'entraînement (6) comporte un deuxième pignon (9), et dans lequel les premier et deuxième éléments d'entraînement peuvent être entraînés par un moteur électrique unique via les premier et deuxième pignons au moyen d'un entraînement à engrenage (11) de l'entraînement à vis télescopique (10).

5. Entraînement à vis télescopique (10) selon l'une des revendications 1 à 4, **caractérisé en ce que** le deuxième filetage du deuxième élément télescopique (2) est un filetage externe qui est formé sur un côté externe de la douille externe (12) du deuxième élément télescopique (2).

6. Entraînement à vis télescopique (10) selon l'une des revendications 1 à 5, **caractérisé en ce que** le troisième élément télescopique (3) est constitué comme une douille de piston.

7. Entraînement à vis télescopique (10) selon l'une des revendications 1 à 6, **caractérisé en ce que** le troisième élément télescopique (3) est bloqué en rotation par rapport au premier élément télescopique (1).

8. Entraînement à vis télescopique (10) selon la revendication 7, **caractérisé en ce que** le premier élément télescopique (1) est réalisé comme une douille fixe, dans lequel l'entraînement à vis télescopique (10) comporte en outre une douille médiane (16) qui entoure radialement au moins partiellement le deuxième élément télescopique (2) et montée de manière coulissante le long de l'axe (4) de l'entraînement à vis télescopique (10) par rapport au premier élément télescopique (1) réalisé comme une douille fixe, dans lequel le troisième élément télescopique (3) est également réalisé comme une douille et est monté de manière coulissante le long de l'axe (4) de l'entraînement à vis télescopique (10) par rapport à la douille médiane (16), et dans lequel un dispositif anti-rotation est présent aussi bien entre le premier élément télescopique (1) et la douille médiane (16) qu'entre la douille médiane (16) et le troisième élément télescopique (3).

9. Entraînement à vis télescopique (10) selon l'une des revendications 1 à 8, **caractérisé en ce que** le premier élément télescopique (1) est l'élément télescopique le plus externe de l'entraînement à vis télescopique (10) et présente un diamètre externe inférieur à 50 mm, et préférablement inférieur à 25 mm.

10. Entraînement à vis télescopique (10) selon l'une des revendications 1 9, **caractérisé en ce que** tous les composants de l'entraînement à vis télescopique (10) qui comportent un filetage sont réalisés en céramique à surface polie, en particulier en céramique à oxyde de zirconium ou d'aluminium.
